# FASCICULE DE BREVET EUROPEEN

(11) **EP 1 861 344 B1**
(45) Date de publication et mention de la délivrance du brevet: **10.12.2008**
(21) Numéro de dépôt: 06726084.4
(22) Date de dépôt: 14.03.2006
(51) Int. Cl.: C07C 6/12, C07C 7/00

(54) **PROCEDE DE PRODUCTION COMBINEE DE PARAXYLENE ET DE BENZENE DE PRODUCTIVITE AMELIOREE**
VERFAHREN ZUR KOMBINIERTEN HERSTELLUNG VON PARAXYLOL UND BENZOL MIT VERBESSERTER PRODUKTIVITÄT
METHOD FOR COMBINED PRODUCTION OF PARAXYLENE AND BENZENE WITH IMPROVED PRODUCTIVITY

(30) Priorité: 16.03.2005 FR 0502621
(43) Date de publication de la demande: 05.12.2007
(73) Titulaire: Institut Français du Pétrole, 92852 Rueil Malmaison Cedex (FR)
(72) Inventeur: HOTIER, Gérard, F-92500 Rueil-Malmaison (FR); SEO, IL, Kim, 1614-14 Sinjeong-Dong, Nam-ku, Ulsan-si (KR)
(86) Numéro de dépôt international: PCT/FR2006/000557
(87) Numéro de publication internationale: WO 2006/097618

(56) Documents cités:
- US-A- 5 401 476
- US-A- 5 866 740
- US-A- 6 004 452

## Description

### Domaine de l'invention:

La production de mondiale de paraxylène est en constante augmentation depuis trente ans à un rythme annuel moyen de 5 à 6%. Les utilisations classiques du paraxylène (PX) sont les productions d'acide téréphtalique, d'anhydride phtalique et de résines polyéthylène téréphtalate, pour fournir des textiles synthétiques, des cassettes audio et vidéo et plus généralement des matières plastiques. Le benzène est un autre intermédiaire pétrochimique ayant de très nombreux usages, par exemple la production d'alkylbenzènes linéaires utilisés dans les lessives, la production de cyclohexane. L'invention concerne un procédé amélioré de production combinée de paraxylène et de benzène à partir d'une première charge aromatique comprenant des xylènes et d'une seconde charge de toluène.

### Art antérieur:

Le paraxylene (PX) est typiquement présent dans des coupes aromatiques en C8 (à 8 atomes de carbone) de réformage catalytique et/ou de vapocraquage. La production de paraxylène s'entend comme la séparation de paraxylène à haute pureté, supérieure à 99%, et par exemple d'au moins 99,7% poids, à partir d'une telle coupe complexe d'hydrocarbures en C8, comprenant éventuellement des quantités mineures d'hydrocarbures aromatiques en C7 et/ou C9 et des quantités mineures d'hydrocarbures non aromatiques, par exemple des naphtènes en C8.

On connaît également deux procédés, ou étapes de procédé additionnelles permettant d'accroître la quantité de paraxylène initialement comprise dans une coupe aromatique:
a) d'une part l'isomérisation convertissant typiquement l'orthoxylène et le métaxylène en un mélange de xylènes proche de l'équilibre thermodynamique. De préférence on utilise une isomérisation transformant également l'éthylbenzène. On connaît déjà dans l'art antérieur deux types principaux d'une telle isomérisation:
   - l'isomérisation "convertissante" transformant en grande partie l'éthylbenzène également en un mélange de xylènes, tel que décrite par le demandeur dans le brevet US 6,369,287. On utilise en général un catalyseur à base de zéolithe (par exemple de type EUO), comprenant du platine et fonctionnant en présence d'hydrogène.
   - l'isomérisation déalkylante, utilisant fréquemment un catalyseur comprenant de la zéolithe ZSM 5 fonctionnant en présence d'hydrogène, et transformant en partie notable ou importante l'éthylbenzène en benzène et éthylène (typiquement hydrogéné en éthane), tel que décrite par exemple dans les brevets US 4,163,028; US4,132,790; et US 4,899,011. On définira conventionnellement une isomérisation comme déalkylante si plus de 30% de l'éthylbenzène qui est converti est déalkylé en benzène. On obtient souvent 70% de transformation par déalkylation.
b) d'autre part la disproportionation (ou dismutation) de toluène en xylènes et benzène. On pourra notamment se référer aux brevets US 4,052,476; US 4,007,231; US 4;011,276; et à l'article "Selective toluène disproportionation process proven at italian refinery" (un procédé de disproportionation sélective de toluène prouvé dans une raffinerie italienne), Gorra F., Breckenbridge L.L., Guy W.M., Sailor R.A., Oil and Gas Journal V90, 60-67 (1992). » concernant la disproportionation sélective de toluene en paraxylène par un procédé appelé MSTDP, ou PX max. On peut également se référer aux brevets EP 26962; US 4,260,843; US 4,274,982; US 4,908,342; US 5, 173,461, et à la demande WO 93/17.987.

L'invention, qui met typiquement en oeuvre ces deux étapes de procédé pour accroître la production totale de paraxylène peut utiliser l'un quelconque des procédés connus de l'art antérieur pour effectuer l'isomérisation et la disproportionation, et n'est pas limitée à l'un ou certains de ces procédés. Toutefois, de façon nettement préférée, on utilise une disproportionation sélective de toluène.

On peut utiliser par ailleurs une isomérisation déalkylante, l'isomérisation convertissante (en xylènes) étant souvent préférée lorsque la charge en C8 aromatiques est en quantité insuffisante.

Les deux techniques, ou procédés de production (en fait de séparation) de paraxylène couramment utilisées, seules ou conjointement, sont l'adsorption en lit mobile simulé (SMB) et la cristallisation.

La première de ces techniques de séparation bien connues est l'adsorption en lit mobile simulé au moyen d'un adsorbant présentant une sélectivité d'adsorption entre le paraxylène et les autres xylènes. On pourra notamment se référer aux brevets US 2,985,589 (contrecourant simulé) ou US 4,402,832 (co-courant simulé).

L'installation d'adsorption comprend typiquement au moins une, et souvent 2 colonnes chromatographiques comprenant chacune une pluralité de lits qui sont interconnectés en boucle fermée. Il y a typiquement au moins deux points d'injection, l'un pour la charge et l'autre pour un désorbant, et au moins deux points de soutirage, l'un pour un extrait E* typiquement riche en PX et l'autre pour un raffinat R* typiquement pauvre ou exempt de PX. Ces 4 points d'injection/soutirage, dont la position varie séquentiellement au cours du temps, délimitent alors 4 zones de fonctionnement de la ou des colonnes d'adsorption.

De nombreuses variantes sont cependant possibles:
- On peut mettre en oeuvre une injection supplémentaire, d'un reflux riche en PX.
- On peut également utiliser un fonctionnement à 5 zones d'adsorption, voire plus, en ne soutirant pas un seul raffinat R*, mais 2 raffinats R1* et R2*. Dans un tel cas le raffinat R1*, ou raffinat intermédiaire, contient typiquement de l'éthylbenzène, et le raffinat R2* contient typiquement de l'orthoxylène (OX) et du métaxylène (MX), la zone entre le soutirage de R1* et celui de R2* étant alors une zone d'adsorption d'éthylbenzène. Ce fonctionnement à deux raffinats R1*, R2* est notamment décrit dans les brevets US 6,838,588; US 6,841,714 et US 6,828,470.

Ces variantes, ainsi que d'autres connues de l'art antérieur, peuvent être mises en oeuvre séparément ou conjointement dans le cadre de l'invention, et l'invention n'est pas liée ni limitée à l'une ou plusieurs d'entre elles.

La seconde technique de séparation bien connue est la cristallisation, qui fournit des cristaux de PX à haute pureté et une liqueur mère ML comprenant du PX de relativement basse pureté. La charge de cristallisation peut typiquement être un effluent d'une unité d'adsorption en lit simulé (on parle alors de technologie hybride) ou un effluent d'une unité de disproportionation de toluène. Il peut y avoir une ou deux zones de cristallisation successives voire plus, éventuellement à des températures différentes (par exemple à haute température [-25°C / +10°C] et basse température [-50°C / -70°C]). On peut aussi mettre en oeuvre une cristallisation partielle de la liqueur mère et/ou une refusion partielle de cristaux. On pourra notamment se référer aux brevets US 3,177,255; US 3,467,724; FR-2 739 375: Ce dernier brevet du demandeur décrit par ailleurs un enchaînement particulier utilisé avantageusement selon l'invention, consistant à mettre en parallèle de l'unité de séparation en lit mobile simulé une disproportionation sélective de toluène suivie par une cristallisation. On peut alors avoir un avantage supplémentaire apporté par un recyclage de la liqueur mère de cristallisation vers la charge de l'unité en lit mobile simulé opéré avec le toluène comme désorbant, afin d'économiser une colonne à distiller.

Dans une cristallisation, il y a typiquement 3 étapes: 1) fabrication de cristaux (tubes réfrigérants raclés, mélange et détente avec évaporation de CO2 ou de propane, film tombant); 2) filtration des cristaux en suspension; 3) récupération et lavage des cristaux pour éliminer la liqueur mère résiduelle.

On peut notamment, selon l'invention, utiliser un cristalliseur statique faisant appel à la technique de cristallisation en film tombant. Ce type d'équipement permet en général de diviser la teneur en impuretés du paraxylène par un facteur compris entre 10 et 15. On peut utiliser plusieurs modules en parallèle et/ou en série. Lorsqu'on traite des xylènes de disproportionation sélective de toluène, la teneur de la charge en PX est typiquement d'environ 85% poids. On peut alors produire dans un premier module du PX de pureté typique comprise entre 98,5% et 99%, et obtenir dans un second module en série une pureté de 99,85% à 99,9%. La liqueur mère produite au second module peut être recyclée au premier module.

Ces variantes et technologies, ainsi que d'autres connues de l'art antérieur (par exemple des cristalliseurs utilisant une filtration et/ou une centrifugation), peuvent être mises en oeuvre séparément ou conjointement dans le cadre de l'invention, et l'invention n'est pas liée ni limitée à l'une ou plusieurs d'entre elles.

Ces deux procédés de séparation du paraxylene à partir d'un mélange de C8 aromatiques que sont la séparation par chromatographie en lit mobile simulé et la cristallisation peuvent être utilisés soit seuls soit en combinaison, comme cela est décrit dans un article du demandeur intitulé « Paraxylene Production with the Eluxyl Process : Debottlenecking with Hybrid Eluxyl » (Production de paraxylène avec le procédé Eluxyl: Dégoulottage avec Eluxyl hybride) par G.Hotier et A.Méthivier Mars 2002 proceedings du congrés de l'AICHE.

Pour satisfaire la demande toujours croissante en paraxylène les pétrochimistes cherchent à produire une quantité maximale de paraxylène dans les unités existantes, en optimisant la conduite du procédé (US 6 004 452). Au dela, ils ont le choix entre des expansions de capacité, soit par dégoulottage d'unités existantes, soit au moyen d'unités neuves très onéreuses produisant typiquement au moins 300 000 t/an et de préférence au moins 450 000 t/an de PX.

Les opérations de dégoulottage de l'art antérieur nécessitent typiquement la modification d'éléments principaux des installations existantes, notamment de la partie SMB, ce qui induit un coût notable.

Il existe également un autre problème, rencontré fréquemment par les pétrochimistes, qui tend à limiter la capacité de production: Ce problème résulte du besoin de maintenir l'unité de chromatographie en lit mobile simulé en production le plus longtemps possible avec une pureté de paraxylene supérieure à 99,7%. Lorsque le tamis moléculaire (adsorbant) placé dans l'unité de séparation en lit mobile simulé est neuf les performances typiques de l'unité sont une pureté de l'ordre de 99.85% et un rendement de l'ordre de 97,5%. Or on constate souvent pour les unités récentes de grande taille, à iso productivité et iso rendement, une perte annuelle de pureté allant de 0,025% à 0,1%, du fait du vieillissement de l'adsorbant. Certains producteurs ont du changer leur tamis moléculaire jusqu'à 3 fois en 6 ans. Pour pouvoir continuer à opérer leur unité avec la pureté requise de 99,7% il a parfois été nécessaire de laisser chuter le rendement en dessous de 80%, ou de réduire fortement le débit de charge en attendant la livraison d'un nouvel inventaire de tamis moléculaire neuf.

Ce problème n'est pas résolu de façon satisfaisante dans l'art antérieur, et il existe donc un intérêt marqué pour un procédé ou une solution technique qui permettrait de prolonger les opérations plusieurs mois de manière à pouvoir coïncider avec les créneaux d'arrêts planifiés.

### Description sommaire de l'invention:

Un premier objet de l'invention est un procédé amélioré de production de paraxylène permettant d'augmenter la production de paraxylène, en opérant différemment des unités existantes.

Un deuxième objet est un procédé permettant de limiter la chute de production résultant du vieillissement naturel et/ou accidentel de l'adsorbant au cours du temps.

Un troisième objet est est un procédé permettant de réaliser un dégoulottage, notamment d'environ 20 à 25% de capacité supplémentaire, avec des modifications limitées d'unités existantes, typiquement en réutilisant tous les équipements principaux et en ajoutant un nombre limité d'équipements.

Le procédé selon l'invention utilise typiquement une production de PX de pureté élevée utilisant en parallèle une adsorption en lit mobile simulé SMB, et une cristallisation d'un effluent de disproportionation sélective de toluène.

L'une des caractéristiques essentielles de l'invention est de substituer, lorsque le fonctionnement du SMB se dégrade du fait du vieillissement de l'adsorbant, une fraction Ea de l'extrait distillé E à la charge initiale de cristallisation, pour produire un paraxylène de cristallisation pratiquement pur et compenser ainsi la baisse de pureté au cours du temps de l'extrait E du SMB, dont la pureté peut alors descendre en dessous de la valeur requise. Les unités d'adsorption et de cristallisation fonctionnent alors partiellement en série (et non totalement en série comme dans la configuration hybride).

On recycle alors avantageusement les xylènes de disproportionation sélective riches en PX au SMB, ce qui améliore son fonctionnement et permet d'accroître son débit d'alimentation. La cristallisation, alimentée par un courant de pureté élevée a également un fonctionnement amélioré et peut opérer à débit fortement augmenté. Le raffinat d'adsorption est typiquement isomérisé puis recyclé.

L'invention permet de maintenir une production élevée de PX et de benzène malgré le vieillissement de l'adsorbant.

La mise en oeuvre préférée de l'invention utilise donc les quatre unités de base: adsorption (SMB), cristallisation, isomérisation, disproportionation sélective. Ces 4 unités sont déjà mises en oeuvre dans certains complexes pétrochimiques.

Dans certains de ces complexes, il existe déja une unité de disproportionation non selective du toluène en benzène et xylènes. Pour transformer cette unité en unité de disproportionation sélective, il suffit généralement de changer de catalyseur. Il en résulte un changement favorable de composition de l'effluent qui devient riche en PX.

Conventionnellement, selon l'invention, on parlera de "disproportionation sélective" lorsque l' effluent de disproportionation a une concentration d'au moins 50% poids en paraxylène à l'intérieur de la coupe C8 de disproportionation (typiquement, on obtient environ 85%). Le caractère sélectif (vers le PX) dépend essentiellement du choix du catalyseur.

Si le complexe ne comprend pas de cristallisation, il faut alors aussi installer une telle unité, typiquement adaptée au débit de l'effluent fractionné (coupe C8) de disproportionation sélective. Dans tous les cas, le dégoulottage que permet l'invention ne requiert pas un investissement lourd tel que l'installation d'une nouvelle unité d'adsorption SMB ou une modification profonde de l'unité SMB existante.

### Description détaillée de l'invention:

Dans ce qui suit on désigne par cycle de fonctionnement du procédé une période de production de paraxylène dans laquelle l'adsorbant (tamis d'adsorption du SMB) est neuf en début de cycle, et ou l'on arrête l'installation SMB en fin de cycle pour remplacer cet adsorbant, qui est alors usé, par de l'adsorbant neuf. Il y a donc un vieillissement continu de l'adsorbant entre le début et la fin d'un cycle.

L'invention propose un procédé de production combinée de paraxylène de haute pureté HPPX et de benzène à partir d'une première charge comprenant principalement des xylènes et de l'éthylbenzène, et d'une deuxième charge comprenant principalement du toluène, dans une installation comprenant au moins:
- une unité de disproportionation sélective de toluène en benzène et xylènes,
- une unité de cristallisation de paraxylène,
- une unité SMB d' adsorption en lit mobile simulé comprenant au moins une colonne chromatographique contenant une pluralité de lits d'un adsorbant ayant une sélectivité différente pour le paraxylène, l'éthylbenzène, le métaxylène et l'orthoxylène, cette colonne utilisant un désorbant,
dans lequel :
a) Pendant la totalité d'au moins un cycle de fonctionnement du procédé,
   - on sépare par adsorption en lit mobile simulé une charge d'adsorption comprenant une fraction au moins de ladite première charge dans ladite unité SMB d' adsorption en lit mobile simulé pour produire un extrait E* riche en paraxylène et au moins un raffinat (R*, R2*) pauvre en paraxylène et contenant de l'orthoxylène et du métaxylène,
   - on distille ledit raffinat (R*, R1*, R2*) pour récupérer un raffinat distillé (R, R1, R2) sensiblement exempt de désorbant,
   - on distille l'extrait E* pour récupérer un extrait distillé E, sensiblement exempt de désorbant,
b) Pendant une fraction initiale IF dudit cycle de fonctionnement du procédé, on produit du paraxylène de haute pureté HPPX comprenant sensiblement la totalité de l'extrait distillé E, l'unité de cristallisation étant pendant ladite fraction initiale IF, soit arrêtée, soit alimentée essentiellement par une partie au moins d'un courant de disproportionation en C8 comprenant essentiellement des xylènes et plus de 50% poids de paraxylène, issu des effluents de conversion de ladite deuxième charge dans ladite unité de disproportionation sélective,
c) Pendant une fraction finale FF dudit cycle de fonctionnement du procédé,
   - on convertit ladite deuxième charge dans l'unité de disproportionation sélective de toluène en benzène et xylènes, pour produire du benzène et un courant de disproportionation en C8 comprenant essentiellement des xylènes et plus de 50% poids de paraxylène,
   - on intègre dans la charge d'adsorption la plus grande partie ou la totalité dudit courant de disproportionation en C8,
   - on divise l'extrait distillé E en une première fraction Ea et une deuxième fraction complémentaire Eb,
   - on alimente alors l'unité de cristallisation essentiellement par Ea, pour produire d'une part du paraxylène PX1 de pureté augmentée, et d'autre part une liqueur mère ML,
   - et l'on mélange PX1 et ladite deuxième fraction complémentaire Eb pour produire le paraxylène de haute pureté HPPX.

Typiquement, la fraction finale du cycle de fonctionnement du procédé est la partie du cycle complémentaire de la fraction initiale du cycle de fonctionnement.

Généralement, au cours de la fraction initiale IF du cycle de fonctionnement, le paraxylène de haute pureté produit HPPX est constitué par la totalité de l'extrait distillé E. Ce paraxylène est alors typiquement à la pureté requise, très généralement au moins 99,7%, par exemple au moins 99,8%.

Lorsque la pureté de E diminue et tend à approcher la valeur limite acceptable, du fait du vieillissement de l'adsorbant, on envoie une fraction Ea du courant E vers la cristallisation. Comme la charge de cristallisation est alors déjà à pureté élevée (comprise typiquement entre 99,4 et 99,7%), le paraxylène de cristallisation obtenu est pratiquement pur (plus de 99,8% et typiquement plus de 99,9%) et permet de compenser le défaut de pureté du courant Eb intégré directement dans HPPX. Ea représente typiquement entre 10% et 70%, et généralement entre 15% et 45% poids de E.

Typiquement, au cours de ladite fraction finale FF du cycle de fonctionnement, la pureté de PX1 est d'au moins 99,8%, celle de Ea et Eb est inférieure à 99,7% et celle de HPPX est d'au moins 99,7%.

Selon une première variante caractéristique préférée du procédé, au cours de la fraction initiale IF du cycle de fonctionnement l'unité de cristallisation est alimentée par une charge de cristallisation constituée par une partie au moins ou la totalité du courant de disproportionation en C8. L'unité de cristallisation est alors en service, alimentée par des C8 de disproportionation de concentration en paraxylène typiquement voisine de 85% poids. Elle produit du paraxylène PX1 de concentration typique d'au moins 99,7% qui est additionné à l'extrait distillé E pour constituer le paraxylène de haute pureté HPPX. Lorsqu'on bascule en mode de fonctionnement final (FF), on bascule typiquement la charge antérieure de cristallisation (C8 de disproportionation, de concentration typique voisine de 85% poids en PX) vers l'adsorption en lit mobile simulé SMB. Ceci enrichit en PX la charge du SMB, ce qui améliore son fonctionnement (rendement et/ou pureté).

Selon une deuxième variante caractéristique du procédé, au cours de la fraction initiale IF du cycle de fonctionnement, l'unité de cristallisation n'est pas en service. Cette variante est avantageusement utilisée lorsque le marché du benzène est excédentaire ou que la valorisation médiocre du benzène ne justifie pas à elle seule les coûts opératoires cumulés de disproportionation et de cristallisation. La situation est modifiée en fin de cycle car l'alimentation de C8 de disproportionation, de concentration typique élevée en PX (ex: 85%) enrichit en PX la charge du SMB et permet donc de produire plus de PX et/ou d'améliorer sa pureté en sortie du SMB.

De façon générale, le fonctionnement de l'ensemble disproportionation/cristallisation permet à la fois de produire du benzène et de produire du paraxylène supplémentaire et/ou de pureté supérieure, ce qui permet d'allonger la durée du cycle en maintenant une production donnée.

Typiquement, le débit de la charge de cristallisation est notablement plus important pendant la fraction finale FF du cycle que pendant la fraction initiale IF du cycle. Ce débit de la charge de cristallisation est typiquement au moins accru de 50%, ou même doublé voire plus pendant la fraction finale FF du cycle par rapport à la fraction initiale IF du cycle. Ceci résulte de la très grande pureté de la charge de cristallisation: Elle permet notamment souvent de fonctionner avec 2 étages de cristallisation en parallèle au lieu de 2 étages en série. Par ailleurs on peut fréquemment éviter de réaliser une refonte partielle du PX cristallisé, celui-ci étant directement à une pureté extrême, ce qui économise une partie du temps de fonctionnement et augmente donc la capacité.

Avantageusement, on recycle une partie au moins, ou généralement la totalité de la liqueur mère ML dans la charge d'adsorption, ce qui permet de récupérer du PX supplémentaire.

Généralement, l'installation pour la mise en oeuvre du procédé, qui est un autre objet de l'invention, comprend également au moins une unité d'isomérisation alimentée par le raffinat distillé (R, R1, R2), et au moins une partie de l'effluent d' isomérisation (de sa fraction en C8) est recyclée et intégrée dans la charge d'adsorption.

Selon une autre variante caractéristique de l'invention, la colonne chromatographique (ou l'unité SMB à 2 colonnes) produit un premier raffinat R1 * comprenant de l'éthylbenzène, de l'orthoxylène et du métaxylène, et un second raffinat R2* sensiblement exempt d'éthylbenzène et comprenant de l'orthoxylène et du métaxylène, on alimente le premier raffinat R1, après distillation pour éliminer sensiblement le désorbant qu'il contient, dans une première unité d'isomérisation, en phase gazeuse, pour convertir une partie au moins de l'éthylbenzène en benzène et/ou xylènes, on alimente le second raffinat R2, après distillation pour éliminer sensiblement le désorbant qu'il contient, dans une seconde unité d'isomérisation, en phase liquide, et on recycle au moins une partie de chacun des effluents d'isomérisation dans la charge d'adsorption.

L'utilisation d'une isomérisation déalkylante est d'ailleurs plus générale (avec 1 ou 2 raffinats). On alimente alors le raffinat distillé (R, R1) dans une unité d'isomérisation déalkylante pour convertir une partie au moins de l'éthylbenzène en benzène, et l'unité produit alors du benzène dont une partie provient de l'unité de disproportion de toluène et une autre partie provient de l'unité d'isomérisation déalkylante.

Lorsque l'on utilise deux raffinats et deux unités d'isomérisation, la première unité d'isomérisation (de R1), en phase gazeuse, est souvent une unité d'isomérisation déalkylante pour convertir une partie au moins de l'éthylbenzène en benzène, et la seconde unité d'isomérisation (de R2), en phase liquide, est typiquement une unité d'isomérisation convertissante et non déalkylante pour convertir la plus grande partie au moins de l'orthoxylène et du paraxylène en un mélange de xylènes comprenant du PX (typiquement proche de l'équilibre thermodynamique), et l'on produit du benzène dont une partie provient de l'unité de disproportion de toluène et une autre partie provient de l'unité d'isomérisation déalkylante. la seconde unité d'isomérisation en phase liquide permet donc d'augmenter la quantité de paraxylène. Lorsqu'on veut maximiser cette production de PX, la première unité d'isomérisation peut également être convertissante et non déalkylante.

Typiquement, on ne recycle pas vers l'unité d'adsorption la totalité des effluents d'isomérisation. Généralement, on sépare par des distillations les fractions différentes de l'isomérat essentiellement aromatique (notamment le benzène et les légers comprenant notamment le propane, le butane), et l'on ne recycle que la coupe essentiellement aromatique en C8.

Souvent, la première charge est préalablement distillée dans une colonne de distillation des xylènes, d'ou l'on soutire une fraction de tête comprenant la majeure partie du métaxylène, du paraxylène, de l'éthylbenzène et au moins une partie de l'orthoxylène, fraction de tête qui est intégrée à la charge d'adsorption, et une fraction de fond comprenant des hydrocarbures en C9-C10 et/ou la partie restante de l'orthoxylène.

### Description des figures:

La figure 1 représente un schéma simplifié d'une installation pour la mise en oeuvre du procédé selon l'invention montrant la circulation typique des différents courants en début d'un cycle de production (mode de circulation "début de vie du tamis", pendant la fraction IF du cycle.
La figure 2 représente un schéma simplifié d'une installation pour la mise en oeuvre du procédé selon l'invention montrant la circulation typique des différents flux en fin d'un cycle de production (mode de circulation typique "fin de vie" pendant la fraction FF du cycle).

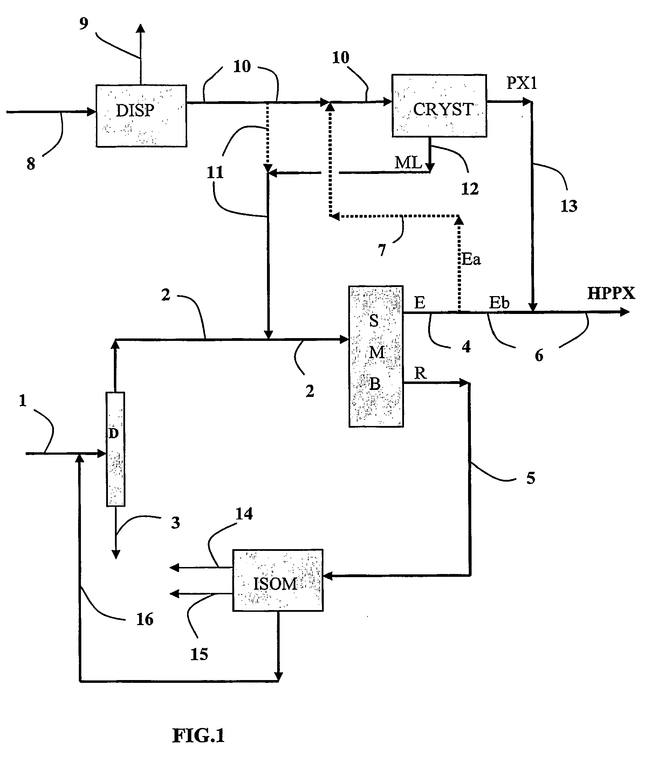

On se réfère maintenant à la figure 1 (début de cycle):

Le schéma de la figure 1 représente une distillation initiale (D) de la charge, une unité (SMB) d'adsorption en lit mobile simulé, une unité (ISOM) d'isomérisation, une unité (DISP) de disproportionation sélective de toluène, et une unité (CRYST) de crystallisation de paraxylène PX.

La première charge aromatique, comprenant des xylènes et typiquement une fraction mineure en C9/C10 rentre par la ligne 1 dans la distillation (D). Elle est mélangée avec un isomérat circulant par la ligne 16, puis fractionnée en une coupe de tête sortant par la ligne 2, comprenant la majeure partie du PX, MX, de l'éthylbenzène EB, et au moins une partie de OX. En fond de colonne sort par la ligne 3 un flux d'hydrocarbures en C9/C10 et la partie restante de l'OX. La coupe de tête, additionnée par des courants provenant de la ligne 11 alimente l'adsorption en lit mobile simulé (SMB), typiquement à contrecourant simulé et au moins 4 zones d'adsorption. Le désorbant utilisé (dont l'entrée dans le SMB n'est pas représentée) peut être notamment du toluène ou du paradiéthylbenzène, ou tout autre solvant sélectif. Il sort de l'unité SMB un extrait E*, qui est distillé (de façon non représentée) pour éliminer le solvant, qui est recyclé, et produire un extrait distillé E par la ligne 4. Il sort également un raffinat R*, qui est distillé (également de façon non représentée) pour éliminer le solvant, qui est recyclé, et produire un raffinat distillé R par la ligne 5.

L'extrait distillé E, en début de cycle, circule typiquement en entier dans la ligne 6 (E= Eb; Ea=0; et la ligne 7 n'est pas alimentée). Cet extrait E a généralement, lorsque l'adsorbant est neuf une pureté de 99,85 et est obtenu avec un rendement de 97%. E est alors mélangé à l'effluent de cristallisation PX1 (paraxylène cristallisé, séparé de la liqueur mère ML, typiquement lavé et refondu, de pureté typique 99,7% à 99,8%) circulant dans la ligne 13. On obtient alors le paraxylène de haute pureté HPPX. On peut aussi éventuellement fonctionner en poussant le débit de charge du SMB pour produire une quantité accrue de PX (extrait E) de pureté juste satisfaisante, par exemple entre 99,7 et 99, 75%.

Le raffinat distillé R, contenant OX, MX et EB alimente l'isomérisation, typiquement déalkylante (ISOM) par la ligne 5, produisant après distillation une petite quantité de produits légers incondensables par la ligne 14 et du benzène par la ligne 15. Du toluène peut aussi être évacué en mélange par la ligne 15 ou par une ligne séparée non référencée. Des C8 contenant du paraxylène (isomérat) sont recyclés à l'entrée de la colonne D par la ligne 16. Souvent ce recyclage d'isomérat est soumis à un traitement à la terre préalable, non représenté, pour enlever les traces d'oléfines présentes.

Une charge de toluène est par ailleurs alimentée par la ligne 8 dans l'unité (DISP) de disproportionation sélective de toluène en benzène et xylènes (principalement PX). Après distillation (plusieurs colonnes) des effluents, le toluène n'ayant pas réagi est recyclé (par une ligne non représentée), le benzène est évacué par la ligne 9, et une coupe en C8, riche en PX (environ 85% poids) est transférée par la ligne 10 vers l'unité de cristallisation (CRYST). Cette unité produit du PX de haute pureté (suffisante, par exemple entre 99,7% et 99,85%) et une liqueur mère ML qui est recyclée à l'unité (SMB) par les lignes 12 puis 11.

Comme le montre la figure 1, l'installation produit, en début de cycle (adsorbant neuf d'efficacité maximale), du PX de haute pureté HPPX en quantité et/ou pureté maximale(s) provenant en parallèle d'une part de l'unité (SMB), dont la pureté de l'extrait distillé excède alors typiquement ou est voisine de 99,7%, et d'autre part de l'unité de cristallisation. Le débit de PX1 par rapport à celui de E est alors souvent compris entre 8% et 40%, et de préférence entre 10% et 25% de E.

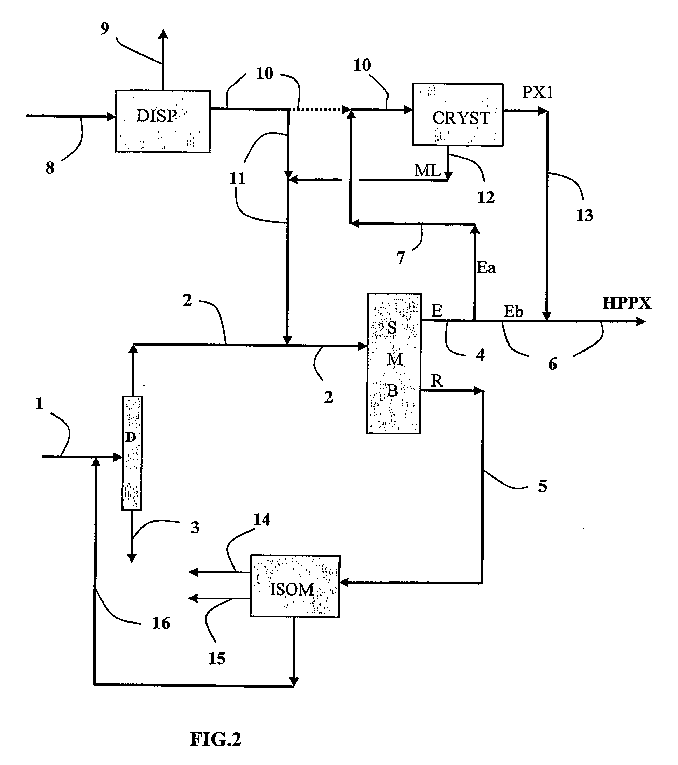

La figure 2 représente la circulation typique des différents courants en fin de cycle, lorsque l'adsorbant a vieilli, possède une efficacité et/ou une sélectivité limitées, se traduisant par une chute (limitée) de la pureté de E en dessous de 99,7% poids, par exemple entre environ 99,45 et 99,7% poids. Dans ce cas, on divise le courant E en un courant Ea recyclé à la cristallisation (CRYST) par la ligne 7, et un courant complémentaire Eb qui continue à former une fraction de HPPX, mais en proportion moindre qu'en début de cycle.

Du fait de la pureté très grande de la charge de cristallisation, le débit de cette charge peut être augmenté fortement, et la pureté de PX1 peut être extrême, par exemple entre 99,9% à 99, 99%, avec un rendement élevé. On produit donc alors du paraxylène de pureté supérieure (PX1), ce qui compense le défaut de pureté de Eb, directement intégré dans HPPX dont la pureté reste d'au moins 99, 7%. De façon à minimiser la consommation de frigories de la cristallisation, on peut augmenter progressivement Ea en fin de cycle, de façon à assurer un débit Ea juste suffisant pour que la pureté de HPPX soit juste celle requise (par exemple 99,7%).

L'invention permet donc de maintenir une pureté cible sans devoir arrêter l'unité SMB, y compris lorsque la pureté de l'extrait de cette unité chute en dessous de la valeur cible.

Du fait de l'envoi de Ea à la charge de cristallisation, une partie ou la totalité de l'effluent en C8 de disproportionation est renvoyé vers l'adsorption (SMB) par la ligne 11. La charge du SMB ayant alors de ce fait une concentration accrue en PX, cela améliore la productivité et/ou la pureté de l'extrait distillé E. Cela permet aussi d'augmenter le débit d'alimentation du SMB.

L'invention permet donc de pouvoir produire de façon maximale, et de maintenir une pureté élevée en fin de cycle, sans devoir réduire sensiblement la production de HPPX.

Par des modifications limitées d'unités existantes, telles qu'un changement de catalyseur de disproportionation et/ou adjonction d'une cristallisation, l'invention permet par ailleurs un dégoulottage notable d'installations existantes qui n'ont pas ces unités, sans avoir à modifier ou doubler l'unité la plus coûteuse (SMB).

De nombreuses variantes peuvent être utilisées par l'homme du métier sans sortir du cadre de l'invention. On peut notamment utiliser une unité (SMB) avec deux raffinats R1* et R2* au lieu d'un, et/ou une unité d'isomérisation non alkylante pour l'éthylbenzène, et/ou diverses configurations d'unités de cristallisation.

On ne sortirait pas non plus du cadre de l'invention si l'on mettait en oeuvre un fonctionnement avec des différences mineures telles que par exemple: envoyer en début de cycle (IF) une fraction mineure de l'extrait vers la cristallisation (en mélange avec la charge principale de xylènes de disproportionation sélective), ou bien envoyer en fin de cycle une fraction mineure de xylènes de disproportionation sélective vers la cristallisation (en mélange avec la charge principale Ea).

### Exemples:

### Exemple 1 selon l'art antérieur.

L'installation du procédé selon l'exemple 1 correspond à celle indiquée à la figure 1, et comprend une unité SMB à un seul raffinat, et une isomérisation déalkylante. Toutefois, les lignes représentées en pointillé sur cette figure (ligne 7 et première partie de la ligne 11) ne sont pas comprises dans cette installation.

Le schéma des flux reste identique, entre le début de cycle et la fin de cycle, mais avec un débit de première charge qui diminue et une production de PX sortie SMB qui diminue notablement en fin de cycle de façon à pouvoir maintenir la pureté minimale requise (99,7%) pour le paraxylène. Le rendement du SMB chute également ainsi que la production de benzène.

Le tableau 1 présente un bilan matière (en tonnes par heure) des différents flux référencés à la figure 1 en début de cycle (adsorbant neuf), et le tableau 2 présente le même bilan matière mais en fin de cycle (adsorbant usé, juste avant son remplacement). On utilise les notations suivantes: PX: paraxylène; OX: orthoxylène; MX: métaxylène; EB: éthylbenzène; T: toluène; BZ: benzène; LPG: C4/C3, et éventuellement des petites quantités de C2 et C1.

**Tableau 1:**

| Début cycle | charge 1 | entrée SMB | R | E | Ea | char ge 2 | sortie DISP | entrée CRYST | PX1 | ML | HPPX | sortie ISOM |
|---|---|---|---|---|---|---|---|---|---|---|---|---|
| EB | 17 | 22,4 | 22,4 | - | - | - | - | - | - | - | - | 5,4 |
| PX | 19,6 | 90,8 | 2,7 | 88,1 - | | - | 17 | 17 | 11,9 | 5,1 | 100 | 66,1 |
| MX | 40 | 177 | 177 | - | - | - | 2 | 2 | - | 2 | - | 135 |
| OX | 20,4 | 90,2 | 90,2 | | - | - | 1 | 1 | - | 1 | - | 68,8 |
| T | - | - | - | - | - | 42 | - (*) | - | - | - | - | - |
| BZ | - | - | - | - | - | - | 20 | - | - | - | - | 12,4 |
| LPG et pertes | - | - | - | - | - | - | 2 | - | - | - | - | 4,6 |
| Total | 97 | 380,4 | 292,3 | 88,1 | 0 | 42 | 42 | 20 | 11,9 | 8,1 | 100 | 292,3 |

| | | | | | | | | | | | | |
|---|---|---|---|---|---|---|---|---|---|---|---|---|
| (*) après recyclage du toluène non converti. La conversion par passe est typiquement de 30%. | | | | | | | | | | | | |

La pureté de l'extrait distillé E est en début de cycle de 99, 85%, avec un rendement élevé (au niveau du SMB) de 97%.

**Tableau 2:**

| Fin de cycle | charge 1 | entrée SMB | R | E | Ea | charge 2 | sortie DISP | entrée CRYS | PX1 | ML | HPPX | sortie ISOM |
|---|---|---|---|---|---|---|---|---|---|---|---|---|
| EB | 13,6 | 17,89 | 17,89 | - | - | - | - | - | - | - | - | 4,29 |
| PX | 15,68 | 78,37 | 6,27 | 72,1 - | | - | 17 | 17 | 11,9 | 5,1 | 84 | 57,59 |
| MX | 32 | 151,53 | 151,53 | - | - | - | 2 | 2 | - | 2 | - | 117,53 |
| OX | 16,32 | 77,26 | 77,26 | | - | - | 1 | 1 | - | 1 | - | 59,94 |
| T | - | - | - | - | - | 42 | - (*) | - | - | - | - | - |
| BZ | - | - | - | - | - | - | 20 | - | - | - | - | 9,88 |
| LPG+ pertes | - | - | - | - | - | - | 2 | - | - | - | - | 3,72 |
| Total | 77,6 | 325.05 | 252,95 | 72,1 | 0 | 42 | 42 | 20 | 11,9 | 8,1 | 84 | 292,3 |

On constate qu'en fin de cycle, du fait du vieillissement de l'adsorbant, on a du baisser de débit d'alimentation de la première charge (et donc de la coupe de tête de la colonne D) d'environ 20% pour maintenir une pureté minimale de l'extrait de 99,7%. Parallèlement, le rendement en PX du SMB a chuté, passant de 97% à 92%. Il en résulte une perte de production globale (HPPX) de 16 %, ce qui est une perte importante. De plus, la diminution de charge entraîne une baisse de la production globale de benzène d'environ 8 %.

### Exemple 2 selon l'invention:

Le fonctionnement en début de cycle est identique à celui de l'art antérieur. Par contre en fin de cycle (par exemple dès que la pureté de l'extrait raffiné E s'approche de 99,7%, à débits de première et seconde charges constants), on remplace l'alimentation de la cristallisation par une fraction Ea de E, et l'on renvoie la coupe C8 de disproportionation vers l'alimentation du SMB, ce qui améliore son fonctionnement du fait de l'enrichissement en PX de la charge globale du SMB. Le débit d'alimentation du SMB peut alors être augmenté, et le bilan matière s'établit alors ainsi:

**Tableau 3:**

| Fin de cycle | charge 1 | entrée SMB | R | E | Ea | charge 2 | sortie DISP | entrée CRYS | PX1 | ML | HPPX | sortie ISOM |
|---|---|---|---|---|---|---|---|---|---|---|---|---|
| EB | 17 | 22,4 | 22,3 | 0,08 | 0,03 | - | - | 0,03 | - | 0,03 | 0,05 | 5,4 |
| PX | 19,6 | 106,8 | 3,2 | 103,64 | 39,86 | - | 17 | 39,86 | 36,01 | 3,85 | 99,78 | 66,4 |
| MX | 40 | 177,6 | 177,4 | 0,22 | 0,09 | - | 2 | 0,09 | - | 0,08 | 0,15 | 135,5 |
| OX | 20,4 | 90,55 | 90,4 | 0,11 | 0,04 | - | 1 | 0,04 | 0,01 | 0,04 | 0,07 | 69,1 |
| T | - | - | - | - | - | 42 | - (*) | - | - | - | - | - |
| BZ | - | - | - | - | - | - | 20 | - | - | - | - | 12,3 |
| LPG+ pertes | - | - | - | - | - | - | 2 | - | - | - | - | 4,6 |
| Total | 97 | 397,35 | 293,3 | 104,05 | 40,02 | 42 | 42 | 40,02 | 36,02 | 4 | 100,05 | 293,3 |

On constate donc que l'invention permet de maintenir en fin de cycle une production identique, sans pertes de paraxylène ni de benzène. Ceci conduit à des gains importants par rapport à l'art antérieur. La pureté du paraxylène HPPX, qui était supérieure à 99,7% en début de cycle est simplement retombée à la valeur requise de 99,7% en fin de cycle.

## Revendications

1. Procédé de production combinée de paraxylène de haute pureté HPPX et de benzène à partir d'une première charge comprenant principalement des xylènes et de l'éthylbenzène, et d'une deuxième charge comprenant principalement du toluène, dans une installation comprenant au moins:
- une unité de disproportionation sélective de toluène en benzène et xylènes,
- une unité de cristallisation de paraxylène,
- une unité SMB d' adsorption en lit mobile simulé comprenant au moins une colonne chromatographique contenant une pluralité de lits d'un adsorbant ayant une sélectivité différente pour le paraxylène, l'éthylbenzène, le métaxylène et l'orthoxylène, cette colonne utilisant un désorbant,
dans lequel :
a) Pendant la totalité d'au moins un cycle de fonctionnement du procédé,
- on sépare par adsorption en lit mobile simulé une charge d'adsorption comprenant une fraction au moins de ladite première charge dans ladite unité SMB d' adsorption en lit mobile simulé pour produire un extrait E* riche en paraxylène et au moins un raffinat pauvre en paraxylène et contenant de l'orthoxylène et du métaxylène,
- on distille ledit raffinat pour récupérer un raffinat distillé (R) sensiblement exempt de désorbant,
- on distille l'extrait E* pour récupérer un extrait distillé E sensiblement exempt de désorbant,
b) Pendant une fraction initiale IF dudit cycle de fonctionnement du procédé, on produit du paraxylène de haute pureté HPPX comprenant sensiblement la totalité de l'extrait distillé E, l'unité de cristallisation étant pendant ladite fraction initiale IF, soit arrêtée, soit alimentée essentiellement par une partie au moins d'un courant de disproportionation en C8 comprenant essentiellement des xylènes et plus de 50% poids de paraxylène, issu des effluents de conversion de ladite deuxième charge dans ladite unité de disproportionation sélective,
c) Pendant une fraction finale FF dudit cycle de fonctionnement du procédé,
- on convertit ladite deuxième charge dans l'unité de disproportionation sélective de toluène en benzène et xylènes, pour produire du benzène et un courant de disproportionation en C8 comprenant essentiellement des xylènes et plus de 50% poids de paraxylène,
- on intègre dans la charge d'adsorption la plus grande partie ou la totalité dudit courant de disproportionation en C8,
- on divise l'extrait distillé E en une première fraction Ea et une deuxième fraction complémentaire Eb,
- on alimente l'unité de cristallisation essentiellement par Ea, pour produire d'une part du paraxylène PX1 de pureté augmentée, et d'autre part une liqueur mère ML,
- et l'on mélange PX1 et ladite deuxième fraction complémentaire Eb pour produire le paraxylène de haute pureté HPPX.

2. Procédé selon la revendication 1, dans lequel ladite fraction finale dudit cycle de fonctionnement du procédé est la partie du cycle complémentaire de ladite fraction initiale du cycle de fonctionnement.

3. Procédé selon l'une des revendications 1 et 2 dans lequel, au cours de ladite fraction initiale IF dudit cycle de fonctionnement, le paraxylène de haute pureté produit HPPX est constitué par la totalité de l'extrait distillé E.

4. Procédé selon l'une des revendications précédentes dans lequel Ea représente entre 20% et 70% de E.

5. Procédé selon l'une des revendications précédentes dans lequel, au cours de ladite fraction initiale IF dudit cycle de fonctionnement, l'unité de cristallisation est alimentée par une charge de cristallisation constituée par une partie au moins ou la totalité dudit courant de disproportionation en C8.

6. Procédé selon l'une des revendications 1 à 4 dans lequel, au cours de ladite fraction initiale IF dudit cycle de fonctionnement, l'unité de cristallisation n'est pas en service.

7. Procédé selon l'une des revendications précédentes, dans lequel, au cours de ladite fraction finale FF dudit cycle de fonctionnement, la pureté de PX1 est d'au moins 99,8%, celle de Ea et Eb est inférieure à 99,7% et celle de HPPX est d'au moins 99,7%.

8. Procédé selon l'une des revendications précédentes, dans lequel le débit de la charge de cristallisation est notablement plus important pendant la fraction finale FF du cycle que pendant la fraction initiale IF du cycle.

9. Procédé selon la revendication 8 dans lequel le débit de la charge de cristallisation est au moins doublé pendant la fraction finale FF du cycle par rapport à la fraction initiale IF du cycle.

10. Procédé selon l'une des revendications précédentes, dans lequel on recycle une partie au moins, ou la totalité de la liqueur mère ML dans la charge d'adsorption.

11. Procédé selon l'une des revendications précédentes, dans lequel ledit raffinat distillé est alimenté dans une unité d'isomérisation, et au moins une partie de cet effluent d' isomérisation est recyclée et intégrée dans la charge d'adsorption.

12. Procédé selon la revendication 11, dans lequel ladite colonne chromatographique produit un premier raffinat R1* comprenant de l'éthylbenzène, un second raffinat R2* sensiblement exempt d'éthylbenzène et comprenant de l'orthoxylène et du métaxylène, et dans lequel on alimente le premier raffinat R1, après distillation pour éliminer sensiblement le désorbant qu'il contient, dans une première unité d'isomérisation, en phase gazeuse, pour convertir une partie au moins de l'éthylbenzène en benzène et/ou xylènes, on alimente le second raffinat R2, après distillation pour éliminer sensiblement le désorbant qu'il contient, dans une seconde unité d'isomérisation, en phase liquide, et on recycle au moins une partie de chacun des effluents d'isomérisation dans la charge d'adsorption.

13. Procédé selon l'une des revendications 11 et 12, dans lequel on alimente ledit raffinat distillé: R ou R1 dans une unité d'isomérisation déalkylante pour convertir une partie au moins de l'éthylbenzène en benzène, et l'on produit du benzène dont une partie provient de l'unité de disproportion de toluène et une partie provient de ladite unité d'isomérisation déalkylante.

14. Procédé selon la revendication 12, dans lequel ladite première unité d'isomérisation, en phase gazeuse, est une unité d'isomérisation déalkylante pour convertir une partie au moins de l'éthylbenzène en benzène, et ladite seconde unité d'isomérisation, en phase liquide, est une unité d'isomérisation convertissante et non déalkylante pour convertir pour convertir le raffinat R2 comprenant de l'orthoxylène et du paraxylène en un mélange de xylènes comprenant du PX, et l'on produit du benzène dont une partie provient de l'unité de disproportion de toluène et une partie provient de ladite unité d'isomérisation déalkylante.

15. Procédé selon l'une des revendications précédentes dans lequel la première charge est préalablement distillée dans une colonne de distillation des xylènes, d'ou l'on soutire une fraction de tête comprenant la majeure partie du métaxylène, du paraxylène, de l'éthylbenzène et au moins une partie de l'orthoxylène, fraction de tête qui est intégrée à la charge d'adsorption, et une fraction de fond comprenant des hydrocarbures en C9-C10 et/ou la partie restante de l'orthoxylène.

## Claims

1. A process for the combined production of high purity para-xylene HPPX and benzene from a first feed principally comprising xylenes and ethylbenzene, and from a second feed principally comprising toluene, in a facility comprising at least the following:
• a unit for the selective disproportionation of toluene to benzene and xylenes;
• a unit for para-xylene crystallization;
• a SMB unit for simulated moving bed adsorption, comprising at least one chromatographic column containing a plurality of beds of an absorbent having a different selectivity for para-xylene, ethylbenzene, meta-xylene and ortho-xylene, said column using a desorbant;
in which:
a) during the whole of at least one operational cycle of the process:
• an adsorption feed comprising at least a fraction of said first feed is separated by simulated moving bed adsorption in said simulated moving bed adsorption unit SMB to produce an extract E* which is rich in para-xylene and at least one raffinate which is depleted in para-xylene and contains ortho-xylene and meta-xylene;
• said raffinate is distilled to recover a distilled raffinate (R) which is substantially free of desorbant;
• the extract E* is distilled to recover a distilled extract E, substantially free of desorbant;
b) during an initial fraction IF of said operational cycle of the process, high purity para-xylene HPPX is produced comprising substantially all of the distilled extract E, the crystallization unit being, during said initial fraction IF, either stopped or essentially supplied with at least a portion of a C₈ disproportionation stream essentially comprising xylenes and more than 50% by weight of para-xylene, derived from effluents from conversion of said second feed in said selective disproportionation unit;
c) during a final fraction FF of said operational cycle of the process;
• said second feed is converted in the unit for selective disproportionation of toluene to benzene and xylenes, to produce benzene and a C₈ disproportionation stream essentially comprising xylenes and more than 50% by weight of para-xylene;
• the largest portion or all of said C₈ disproportionation stream is integrated into the adsorption feed;
• the distilled extract E is divided into a first fraction Ea and a complementary second fraction Eb;
• the crystallization unit is essentially supplied with Ea to produce higher purity para-xylene PX1 and a mother liquor ML;
• and the PX1 and said complementary second fraction Eb are mixed to produce high purity para-xylene HPPX.

2. A process according to claim 1, in which said final fraction of said operational cycle of the process is the part of the cycle which is complementary to the initial fraction of the operational cycle.

3. A process according to claim 1 or claim 2, in which during the initial fraction IF of said operational cycle, the high purity para-xylene HPPX produced is constituted by all of the distilled extract E.

4. A process according to one of the preceding claims, in which Ea represents between 20% and 70% of E.

5. A process according to one of the preceding claims in which, during the initial fraction IF of said operating cycle, the crystallization unit is supplied with a crystallization feed constituted by at least a part or all of the C₈ disproportionation stream.

6. A process according to one of claims 1 to 4, in which during said initial fraction IF of the operational cycle, the crystallization unit is not in service.

7. A process according to one of the preceding claims in which, during said final fraction FF of said operational cycle, the purity of PX1 is at least 99.8%, that of Ea and Eb is less than 99.7%, and that of HPPX is at least 99.7%.

8. A process according to one of the preceding claims, in which the flow rate of the crystallization feed is substantially higher during the final fraction FF of the cycle than during the initial fraction IF of the cycle.

9. A process according to claim 8, in which the flow rate of the crystallization feed is at least doubled during the final fraction FF of the cycle compared with the initial fraction IF of the cycle.

10. A process according to one of the preceding claims, in which at least a part or all of the mother liquor ML is recycled to the adsorption feed.

11. A process according to one of the preceding claims, in which said distilled raffinate is supplied to an isomerization unit and at least a portion of said isomerization effluent is recycled and integrated into the adsorption feed.

12. A process according to claim 11, in which the chromatographic column produces a first raffinate R1* comprising ethylbenzene, and a second raffinate R2* which is substantially free of ethylbenzene and comprises ortho-xylene and meta-xylene, and in which the first raffinate R1, after distillation to substantially eliminate the desorbant it contains, is supplied to a first isomerization unit in the gas phase to convert at least a portion of the ethylbenzene to benzene and/or xylenes and the second raffinate R2, after distillation to substantially eliminate the desorbant it contains, is supplied to a second isomerization unit in the liquid phase, and at least a portion of each of the isomerization effluents is recycled to the adsorption feed.

13. A process according to claim 11 or claim 12, in which said distilled raffinate: R or R1 is supplied to a dealkylating isomerization unit to convert at least a portion of the ethylbenzene to benzene, and benzene is produced a portion of which derives from the toluene disproportionation unit and a portion derives from said dealkylating isomerization unit.

14. A process according to claim 12, in which said first isomerization unit, in the gas phase, is a dealkylating isomerization unit to convert at least a portion of the ethylbenzene to benzene, and said second isomerization unit, in the liquid phase, is a converting and non dealkylating isomerization unit to convert the rafinate R2 comprising ortho-xylene and para-xylene into a mixture of xylenes comprising PX, and benzene is produced a portion of which derives from the toluene disproportionation unit and a portion of which derives from the dealkylating isomerization unit.

15. A process according to one of the preceding claims, in which the first feed is initially distilled in a xylene distillation column, from which an overhead fraction comprising the major portion of meta-xylene, para-xylene, ethylbenzene and at least a portion of the ortho-xylene is withdrawn, which overhead fraction is integrated into the adsorption feed, and a bottom fraction is withdrawn comprising C₉-C₁₀ hydrocarbons and/or the remaining portion of the ortho-xylene.

## Patentansprüche

1. Verfahren zur kombinierten Herstellung von Paraxylol mit hoher Reinheit HPPX und von Benzol aus einer ersten Beschickung, die im Wesentlichen Xylole und Ethylbenzol umfasst, und aus einer zweiten Beschickung, die im Wesentlichen Toluol umfasst, in einer Vorrichtung, die mindestens umfasst:
- eine Einheit zur selektiven Disproportionierung von Toluol in Benzol und Xylole,
- eine Einheit zur Kristallisierung von Paraxylol,
- eine SMB-Einheit zur Adsorption im simulierten Bewegtbett die mindestens eine Chromatographiesäule umfasst, die eine Vielzahl von Adsorptionsbetten enthält, die eine unterschiedliche Selektivität für Paraxylol, Ethylbenzol, Metaxylol und Orthoxylol aufweisen, wobei diese Säule ein Desorbens verwendet,
wobei:
a) während der Gesamtheit mindestens eines Betriebszyklus des Verfahrens,
- durch Adsorption im simulierten Bewegtbett eine Adsorptionsbeschickung getrennt wird, die mindestens eine Fraktion der ersten Beschickung in der SMB-Einheit zur Adsorption im simulierten Bewegtbett umfasst, um einen Extrakt E*, der reich an Paraxylol ist, und mindestens ein Raffinat herzustellen, das arm an Paraxylol ist, und Orthoxylol und Metaxylol enthält,
- das Raffinat destilliert wird, um ein destilliertes Raffinat (R) zu gewinnen, das im Wesentlichen frei von Desorbens ist,
- der Extrakt E* destilliert wird, um einen destillierten Extrakt E zu gewinnen, der im Wesentlichen frei von Desorbens ist,
b) während einer Anfangsfraktion AF des Betriebszyklus des Verfahrens wird Paraxylol mit hoher Reinheit HPPX hergestellt, das im Wesentlichen die Gesamtheit des destillierten Extrakts E umfasst, wobei die Einheit zur Kristallisierung während der Anfangsfraktion AF entweder angehalten ist, oder hauptsächlich mindestens ein Teil eines Stroms zur C8-Disproportionierung eingeleitet wird, der hauptsächlich Xylole und mehr als 50 Gew.-% Paraxylol umfasst, die aus Konversionsabflüssen der zweiten Beschickung in der Einheit zur selektiven Disproportionierung stammen,
c) während einer Endfraktion EF des Betriebzyklus des Verfahrens,
- die zweite Beschickung in der Einheit zur selektiven Disproportionierung von Toluol in Benzol und Xylole umgewandelt wird, um Benzol und einen C8-Disproportionierungsstrom herzustellen, der hauptsächlich Xylole und mehr als 50 Gew.-% Paraxylol umfasst,
- in die Adsorptionsbeschickung der größte Teil oder die Gesamtheit des C8-Disproportionierungsstroms integriert wird,
- der destillierte Extrakt E in eine erste Fraktion Ea und eine zweite komplementäre Fraktion Eb getrennt wird,
- in die Einheit zur Kristallisierung hauptsächlich Ea eingeleitet wird, um einerseits Paraxylol PX1 mit höherer Reinheit und andererseits eine Mutterlauge ML herzustellen,
- und PX1 und die zweite komplementäre Fraktion Eb gemischt wird, um das Paraxylol mit hoher Reinheit HPPX herzustellen.

2. Verfahren nach Anspruch 1, wobei die Endfraktion des Betriebszyklus des Verfahrens der Teil des komplementären Zyklus der Anfangsfraktion des Betriebszyklus ist.

3. Verfahren nach einem der Ansprüche 1 und 2, wobei im Verlauf der Anfangsfraktion AF des Betriebszyklus das produzierte Paraxylol mit hoher Reinheit HPPX in seiner Gesamtheit aus dem destillierten Extrakt E besteht.

4. Verfahren nach einem der vorhergehenden Ansprüche, wobei Ea zwischen 20 % und 70 % von E darstellt.

5. Verfahren nach einem der vorhergehenden Ansprüche, wobei im Verlauf der Anfangsfraktion AF des Betriebszyklus, in die Einheit zur Kristallisierung eine Kristallisierungsbeschickung eingeleitet wird, die mindestens aus einem Teil oder der Gesamtheit des C8-Disproportionierungsstroms besteht.

6. Verfahren nach einem der Ansprüche 1 bis 4, wobei im Verlauf der Anfangsfraktion AF des Betriebszyklus die Einheit zur Kristallisierung nicht in Betrieb ist.

7. Verfahren nach einem der vorhergehenden Ansprüche, wobei im Verlauf der Endfraktion EF des Betriebszyklus die Reinheit von PX1 bei mindestens 99,8 % liegt, die von Ea und Eb kleiner als 99,7 % ist und die von HPPX bei mindestens 99,7 % liegt.

8. Verfahren nach einem der vorhergehenden Ansprüche, wobei die Durchflussgeschwindigkeit der Kristallisierungsbeschickung während der Endfraktion EF des Zyklus deutlich größer ist als während der Anfangsfraktion AF des Zyklus.

9. Verfahren nach Anspruch 8, wobei die Durchflussgeschwindigkeit der Kristallisierungsbeschickung während der Endfraktion EF im Verhältnis zur Anfangsfraktion AF des Zyklus mindestens verdoppelt ist.

10. Verfahren nach einem der vorhergehenden Ansprüche, wobei mindestens ein Teil oder die Gesamtheit der Mutterlauge ML in die Adsorptionsbeschickung recycelt wird.

11. Verfahren nach einem der vorhergehenden Ansprüche, wobei das destillierte Raffinat in eine Einheit zur Isomerisierung geleitet wird, und mindestens ein Teil dieses Isomerisierungsabflusses recycelt und in die Adsorptionsbeschickung integriert wird.

12. Verfahren nach Anspruch 11, wobei die Chromatographiesäule ein erstes Raffinat R1* herstellt, das Ethylbenzol umfasst, ein zweites Raffinat R2*, das im Wesentlichen frei von Ethylbenzol ist und Orthoxylol und Metaxylol umfasst, und wobei das erste Raffinat R1 nach der Destillation eingeleitet wird, um im Wesentlichen das Desorbens zu entfernen, das es enthält, in eine erste Einheit zur Isomerisierung, als gasförmige Phase, um mindestens einen Teil des Ethylbenzols in Benzol und/oder Xylole umzuwandeln, das zweite Raffinat R2 nach der Destillation eingeleitet wird, um im Wesentlichen das Desorbens zu entfernen, das es enthält, in eine zweite Einheit zur Isomerisierung, in flüssiger Phase, und mindestens ein Teil jeder dieser Isomerisierungsabflüsse in die Adsorptionsbeschickung recycelt wird.

13. Verfahren nach einem der Ansprüche 11 und 12, wobei das destillierte Raffinat: R oder R1 in eine Einheit zur desalkylierenden Isomerisierung eingeleitet wird, um mindestens einen Teil des Ethylbenzols in Benzol umzuwandeln, und Benzol hergestellt wird, wovon ein Teil aus der Einheit zur Disproportionierung von Toluol stammt und ein Teil aus der Einheit zur desalkylierenden Isomerisierung stammt.

14. Verfahren nach Anspruch 12, wobei die erste Einheit zur Isomerisierung, in gasförmiger Phase, eine Einheit zur desalkylierenden Isomerisierung ist, um mindestens einen Teil des Ethylbenzols in Benzol umzuwandeln, und die zweite Einheit zur Isomerisierung, in flüssiger Phase, eine Einheit zur umwandelnden und nicht desalkylierende Isomerisierung ist, um das Raffinat R2, das Orthoxylol und Paraxylol umfasst, in ein Gemisch aus Xylolen umzuwandeln, das PX umfasst, und Benzol hergestellt wird, wovon ein Teil aus der Einheit zur Disproportionierung von Toluol stammt und ein Teil aus der Einheit zur desalkylierenden Isomerisierung stammt.

15. Verfahren nach einem der vorhergehenden Ansprüche, wobei die erste Beschickung zuvor in einer Destillationssäule der Xylole destilliert wird, aus der eine Kopffraktion abgezogen wird, die den Großteil des Metaxylols, Paraxylol, Ethylbenzol und mindestens einen Teil des Orthoxylols umfasst, eine Kopffraktion, die in die Adsorptionsbeschickung integriert ist, und eine Bodenfraktion, die Kohlenwasserstoffe mit 9-10 C-Atomen und/oder den verbleibenden Teil des Orthoxylols umfasst.
